# EUROPEAN PATENT SPECIFICATION

(11) **EP 3 801 713 B1**
(45) Date of publication and mention of the grant of the patent: **02.08.2023**
(21) Application number: 19728087.8
(22) Date of filing: 06.06.2019
(51) Int. Cl.: A61M 15/00, A61M 15/08

(54) **INHALER**
INHALATOR
INHALATEUR

(30) Priority: 08.06.2018 EP 18176856
(43) Date of publication of application: 14.04.2021
(73) Proprietor: Presspart Manufacturing S.A., 43720 L'Arboc del Penedes, Tarragona (ES)
(72) Inventor: TORRES MUNIESA, Victor, 43720 L'Arboc del Penedes, Tarragona (ES); HERRERA MARTINEZ, Paloma, 43720 L'Arboc del Penedes, Tarragona (ES); BUSTO SOTIL, Santiago, 43720 L'Arboc del Penedes, Tarragona (ES); VALENCIA PELLISA, David, 43720 L'Arboc del Penedes, Tarragona (ES)
(74) Representative: Uexküll & Stolberg
(86) International application number: PCT/EP2019/064821
(87) International publication number: WO 2019/234173

(56) References cited:
- US-A- 5 060 643
- US-A1- 2006 213 504
- US-A1- 2016 121 060

## Description

### TECHNICAL FIELD

The present invention relates to an inhaler having a hollow inhaler body for retaining an aerosol container with a dispensing valve at a valve end of the container. The inhaler body comprises a first open end sized and arranged to receive the aerosol container with a dispensing valve and a second open end through which a dose of medicament is dispensed as an aerosol. The second open end is sized and arranged to be coupled to the mouth or nasal cavities of a patient. The inhaler body further comprises a nozzle block with an upper portion and a lower portion positioned adjacent to one another. The nozzle block extends along a longitudinal axis from a bottom portion of the inhaler body towards the first open end. The nozzle block is configured such that a dose of a medicament is released upon depression of the dispensing valve of the aerosol container against the nozzle block. The nozzle block has a fluid flow path extending therethrough such that the dose of medicament being released from the valve is passed to the nozzle block and exits the nozzle block as an aerosol in a direction towards the second open end of the inhaler body. The nozzle block is formed as a part separate from the inhaler body and is adapted to be inserted through the first open end of the inhaler body into a receiving portion of the bottom portion.

### BACKGROUND OF THE INVENTION

Inhalers, in particular metered dose inhalers (MDIs), are medication delivery devices which deliver a pharmaceutical formulation including one or more pharmaceutical active compounds to a human or another mammalian patient. Typically, the pharmaceutical formulation is delivered by the MDIs in unit doses in the form of an aerosol. Each actuation of the MDIs delivers one unit dose. The unit dose is expelled by the MDIs and is taken into the body of the patient on inhalation, via the nose or mouth. The pharmaceutical formulation is delivered to or via the respiratory tract, notably to the lungs, of the patient on inhalation. Metered dose inhalers are typically used for the treatment of respiratory infections and disorders including respiratory tract infections, obstructive lung disease, inflammatory lung disease and chronic obstructive pulmonary disease. Asthma treatment is a particularly common use of MDIs

ES 1069857 U discloses such a metered dose inhaler having an angular body with a notably cylindrical vertical part and a lower part. The lower part protrudes forward, constituting an outlet opening forming an angle of greater than 90° with the vertical part. An outlet structure is positioned on the lower base of the vertical part and comprises several holes in order to direct aerosol from a container being received in the vertical part to the outlet opening.

Typically, inhalers are manufactured according to customer specifications. After delivery to the customer the inhalers are then equipped with an aerosol container and brought to the market. Specifications of different customers often only vary with regard to the design of the nozzle block. This is because the design of the nozzle block significantly influences the spray pattern of the aerosol exiting the nozzle towards the second open end of the inhaler body. Moreover, the nozzle block design depends amongst others on the formulation of the medicament to be used in combination with the inhaler. However, the whole inhaler has to be designed and manufactured individually in order to address the needs of every customer which is rather time consuming and expensive.

Moreover, inhalers are preferably manufactured under use of the injection molding technique by which melted plastic particles are injected into moulds where they solidify and take the appearance of an inhaler body. As the geometry of an inhaler body comprises several openings and undercuts its geometry is quite complex. Accordingly, the moulds comprise several parts which are movable relative to one another in order to allow removement of the inhaler body from the mould. Consequently tooling, in particular the moulds used for the injection molding, are very expensive.

US 2016/0121060 A1 relates to a nasal actuator comprising a body, a stem post and fixing means for fixing the stem post in the body. The body comprises a delivery passage for delivery of a medicament and a canister opening for insertion of a canister having a metering valve with a valve stem. The stem post comprises a stem socket for receiving the valve stem of a canister and comprises an orifice for discharging a medicament to the delivery passage. The stem post and body are adapted such that they cooperate to define a transition chamber when the stem post is fixed in the body.

US 2006/213504 A1 relates to an actuator for an inhaler for administering medicament by inhalation and to an inhaler including the same.

It is an object of the present invention to provide a cost effective inhaler which allows a simplified production and addresses individual customer needs.

### SUMMARY OF THE INVENTION

This object is achieved by an inhaler comprising the features of claim 1, preferred embodiments are set out in the dependent claims.

According to the invention the nozzle block has two supporting legs for vertically supporting the nozzle block in the receiving portion. Each of the supporting legs has a lower end with an alignment plate protruding transversely to the longitudinal axis of the nozzle block from the corresponding supporting leg in an outward direction. The alignment plates are configured to rest against corresponding protrusions of the receiving portion and protrude in opposing directions. The corresponding protrusions of the receiving portion oppose each other. The nozzle block has a rib which extends parallel to the supporting legs from the upper portion along the lower portion and is located between the supporting legs. The rib is adapted to engage with a vertically extending groove in the receiving portion.

The supporting legs are suitable to enable a proper stand of the nozzle block in the receiving portion and thus define the standing height of the nozzle block in the receiving portion. The alignment plates together with the rip are suitable to enable a desired orientation of the nozzle block relative to the second open end of the inhaler body. Preferably, when being engaged with the vertical extending groove of the receiving portion, the rib sets the orientation of the nozzle block relative to the second open end in a rotational direction around the longitudinal axis of the nozzle block. The alignment plates optionally provide fixation of the lower portion of the nozzle block in a direction transversally to the longitudinal axis of the nozzle block and towards the second open end of the inhaler body.

In another embodiment of the invention the nozzle block is connected to the bottom portion of the inhaler body via a form fitting connection. Preferably, the nozzle block is inserted into the inhaler body through its first open end and into the receiving portion from above. The form fitting connection may be configured to withstand the forces which occur during operation of the inhaler, in particular during depression and release of the dispensing valve. The form fitting connection allows an easy assembling of the nozzle block and the inhaler body.

In still another embodiment of the invention the nozzle block has an at least partially cylindrical shape, wherein the nozzle block comprises a radially inward extending lower edge which is adapted to engage with a vertically extending engagement member in the receiving portion of the bottom portion to form the form fitting connecting. Optionally the engagement member is a snap-in tongue. Preferably, the snap-in tongue extends vertically from the bottom portion of the inhaler body towards the first open end of the inhaler body. Optionally, upon insertion of the nozzle block into the receiving portion the lower edge of the nozzle block contacts the snap-in tongue, forces the snap-in tongue to bend in a direction transversely to the longitudinal axis upon further depression of the nozzle block and engages with the snap-in tongue upon the snap-in tongue snapping back in its original position. The form fitting connection between the nozzle block and the receiving portion secures the nozzle block in the receiving portion in a vertical direction along the longitudinal axis of the nozzle block. The form fitting connection thus prevents the nozzle block from being removed from the receiving portion towards the first open end of the inhaler body.

Preferably, the receiving portion of the bottom portion has an essentially cylindrical shape, wherein the opposing protrusions extend radially inward. Preferably, the inner shape of the receiving portion is formed corresponding to the outer shape of the nozzle block.

In an embodiment of the invention the fluid flow path of the nozzle block is formed by a sump configured to receive the dispensing valve of the aerosol container, a nozzle configured to aerosolize the dose of medicament being released from the dispensing valve and a channel extending from the sump to the nozzle. In order to ensure a proper functioning of the nozzle block and that the molded nozzle block is dimensionally within the specification, it is, among other dimensions, required to measure the diameter at the transition of the channel to the nozzle (orifice diameter) as well as the length of the channel (jet length).

In another embodiment of the invention the nozzle block is formed of transparent material. This allows visual inspection of the nozzle block after fabrication. In particular, this allows the measurement of the nozzle block by the use of vision systems which don't require a specific handling or physical change (e.g. cutting, adding liquids for increasing contrast etc.). This enables a cheap, fast and inline inspection check for nozzle blocks and thus total batch traceability. Vision systems typically allow to capture a digital image of a part which is to be measured. The measurements are then taken from the image manually or automatically in a subsequent step. Preferably the vision system comprises a camera or any other device which is capable of taking an image of the part to be measured.

Preferably, the lower portion of the nozzle block comprises opposing plane side surfaces which are arranged parallel to one another. The plane side surfaces allow recording of a non-distorted image of the interior of the nozzle block as the rays, in particular light rays, enter and exit the nozzle block preferably at a right angle with regard to the plane side surfaces such that the rays are not deflected. This enables true measurements of the interior of the nozzle block.

In an embodiment of the invention the side surfaces of the lower portion are arranged parallel to a fist axis defined by the channel connecting the sump and the nozzle of the nozzle block and are arranged parallel to a second axis defined by the nozzle block extending from the bottom portion towards the first open end of the inhaler body. Such an arrangement of the side surfaces allows direct measuring of the jet length as well as the orifice in between the channel and the nozzle.

In another embodiment of the invention the inhaler comprises an insert configured to be inserted into the inhaler body through its first open end, wherein the insert exhibits ribs which extend from the first open end towards the bottom portion of the inhaler body and which are adapted to abut the aerosol container upon insertion into the inhaler body. Preferably, the insert is available in different sizes which differ in the size of the ribs in between the inhaler body and the aerosol container and thus in a radial direction, namely in a direction lateral to the second axis. The insert allows the adaption of the inhaler body to receive aerosol containers of different sizes, in particular of different diameters. Thus, by use of inserts having different sizes one single inhaler body may be adapted to accommodate aerosol containers of different sizes, thereby reducing costs of tooling and production costs.

Preferably, the inhaler body has a bottom side, and comprises corrugations thereon. The area at the bottom side of the inhaler body is often used by patients to hold the inhaler during inhalation. As the corrugations allow secure gripping of the inhaler during inhalation an accidently dropping of the inhaler is avoided.

The above object is also achieved by an inhaler comprising the features of claim 11, preferred embodiments are set out in the corresponding dependent claims.

According to still another embodiment of the present invention the nozzle block is formed as a part separate from the inhaler body. The bottom portion of the inhaler body comprises a receiving portion for receiving the nozzle block. The nozzle block is adapted to be inserted through the first open end of the inhaler body into the receiving portion of the bottom portion. The bottom portion and the inhaler body are integrally formed, i.e. as one piece. Preferably, the inhaler body together with its bottom portion is formed via injection molding.

As the nozzle block is formed as a separate part from the inhaler body, the nozzle block can be individually designed according to customer needs, whereas the inhaler body stays the same. Additionally, the nozzle block may be designed as a part being compatible with a range of inhaler bodies having different sizes and/or different shapes. Moreover, the design of the nozzle block can be optimized with regard to the manufacturing process, in particular with regard to the injection molding of the nozzle block, without having to consider producibility of the inhaler body together with the nozzle block in one single mould. This allows cheaper tooling and decreases the production costs. Preferably, the outer shape of the nozzle block, at least in the area which is received by the receiving portion of the inhaler body, stays unchanged in order to ensure a proper fit of the nozzle block in the receiving portion. Optionally, the design changes of the nozzle block for addressing customer needs relate to the fluid flow path extending therethrough.

Preferably, the inhaler according to the present invention may be used as a metered dose inhaler with or without an additional dose counter which may be a mechanical and/or electronic dose counter. Optionally, a mechanical dose counter is located vertically above the nozzle block. Alternatively or additionally an electronic dose counter may be located between a rear side of the inhaler body and the aerosol container or a front side of the inhaler body and the aerosol container. Preferably, the mechanical dose counter is mounted on the bottom portion of the inhaler body and is inserted into the inhaler body through the first open end.

In another embodiment of the present invention the nozzle block is connected to the bottom portion of the inhaler body via a form fitting connection. Preferably the nozzle block is inserted into the inhaler body through its first open end and into the receiving portion from above. The form fitting connection may be configured to withstand the forces which occur during operation of the inhaler, in particular during depression and release of the dispensing valve. The form fitting connection allows an easy assembling of the nozzle block and the inhaler body.

In still another embodiment of the present invention the nozzle block has an at least partially cylindrical shape, wherein the nozzle block comprises a radially outward extending lower edge which is adapted to engage with a slot in the receiving portion of the bottom portion to form the form fitting connecting. Preferably, the lower edge is part of a snap in tongue of the nozzle block. The snap in tongue is configured such that the lower edge snaps into the slot of the receiving portion upon insertion of the nozzle block into the receiving portion. Upon engagement of the lower edge and the slot axial movement of the nozzle block towards the first open end is omitted.

Preferably, the nozzle block has an upper portion, and a lower portion positioned adjacent to one another. Optionally, the upper portion receives the dispensing valve of the aerosol container, whereas the aerosol exits the nozzle block in the area of the lower portion towards the second open end of the inhaler body.

In an embodiment the nozzle block has a supporting leg for vertically supporting the nozzle block in the receiving portion, wherein the supporting leg comprises a T-shaped cross sectional area having a stem and two opposing arms, wherein the stem forms a rib which extends from the upper portion along the lower portion and, wherein the rib is adapted to engage with a vertically extending groove in the receiving portion. The T-shapes cross sectional area extends lateral to an axis defined by the nozzle block extending from the bottom portion towards the first open end of the inhaler body. Upon engagement of the rib and the vertically extending groove in the receiving portion the orientation of the nozzle block around the axis defined by the nozzle block extending from the bottom portion towards the first open end of the inhaler body is set. This ensures that a dose of a medicament being released from the valve of the aerosol container exits the nozzle block as an aerosol in a direction towards the second open end of the inhaler body. The rib of the nozzle block and the groove in the receiving portion are engaged upon insertion of the nozzle block into the receiving portion.

In a preferred embodiment the upper portion comprises opposing recesses, which each have a plane base surface, wherein the base surfaces are arranged parallel to one another. Preferably the base surfaces are arranged parallel to the axis defined by the nozzle block extending from the bottom portion towards the first open end of the inhaler body.

In another embodiment the receiving portion of the bottom portion has an essentially cylindrical shape and comprises opposing protrusions which protrude radially inward. Preferably, the inner shape of the receiving portion is formed corresponding to the outer shape of the nozzle block.

Preferably, the opposing recesses of the nozzle block are configured to engage with the opposing protrusions of the receiving portion upon insertion of the nozzle block into the receiving portion. Optionally, the protrusions form a stop for the recesses and thus keep the nozzle block in an upright position in the receiving portion such that the nozzle block points towards the first open end of the inhaler body. As the engagement of the recesses and the protrusions keeps the nozzle block in the upright position its lower edge is kept in engagement with the slot of the receiving portion. Thus, the engagement of the recesses and the protrusions prevents movement of the nozzle block along the axis defined by the nozzle block extending from the bottom portion towards the first open end of the inhaler body.

According to another embodiment the fluid flow path of the nozzle block is formed by a sump configured to receive the dispensing valve of the aerosol container, a nozzle configured to aerosolize the dose of medicament being released from the dispensing valve and a channel extending from the sump to the nozzle. In order to ensure a proper functioning of the nozzle block and that the molded nozzle block is dimensionally within the specification, it is, among other dimensions, required to measure the diameter at the transition of the channel to the nozzle (orifice diameter) as well as the length of the channel (jet length).

In an embodiment of the present invention the nozzle block is formed of transparent material. This allows visual inspection of the nozzle block after fabrication. In particular, this allows the measurement of the nozzle block by the use of vision systems which don't require a specific handling or physical change (e.g. cutting, adding liquids for increasing contrast etc.). This enables a cheap, fast and 100% inline inspection check for nozzle blocks and thus total batch traceability. Vision systems typically allow to capture a digital image of a part which is to be measured. The measurements are then taken from the image manually or automatically in a subsequent step. Preferably the vision system comprises a camera or any other device which is capable of taking an image of the part to be measured.

Preferably, the lower portion of the nozzle block comprises opposing plane side surfaces which are arranged parallel to one another. The plane side surfaces allow recording of a non-distorted image of the interior of the nozzle block as the rays, in particular light rays, enter and exit the nozzle block preferably at a right angle with regard to the plane side surfaces such that the rays are not deflected. This enables true measurements of the interior of the nozzle block.

In another preferred embodiment the side surfaces of the lower portion are arranged parallel to a first axis defined by the channel connecting the sump and the nozzle of the nozzle block and are arranged parallel to a second axis defined by the nozzle block extending from the bottom portion towards the first open end of the inhaler body. Such an arrangement of the side surfaces allows direct measuring of the jet length as well as the orifice in between the channel and the nozzle.

In another embodiment the inhaler comprises an insert configured to be inserted into the inhaler body through its first open end, wherein the insert has ribs which extend from the first open end towards the bottom portion of the inhaler body and which are adapted to abut the aerosol container upon insertion into the inhaler body. Preferably, the insert is available in different sizes which differ in the size of the ribs in between the inhaler body and the aerosol container and thus in a radial direction, namely in a direction lateral to the second axis. The insert allows the adaption of the inhaler body to receive aerosol containers of different sizes, in particular of different diameters. Thus, by use of inserts having different sizes one single inhaler body may be adapted to accommodate aerosol containers of different sizes, thereby reducing costs of tooling and production costs.

Preferably, the inhaler body has a bottom side, and comprises corrugations thereon. The area at the bottom side of the inhaler body is often used by patients to hold the inhaler during inhalation. As the corrugations allow secure gripping of the inhaler during inhalation an accidently dropping of the inhaler is avoided.

The invention will now be described in connection with exemplary embodiments shown in the figures in which:
- Figure 1: shows a perspective view of an inhaler,
- Figure 2: shows a sectional side view of the inhaler,
- Figure 3: shows a perspective view of a nozzle block,
- Figure 4: shows a front view of the nozzle block of Figure 3,
- Figure 5: shows a perspective rear view of the nozzle block of Figure 3,
- Figure 6A: shows a sectional top view along the line A-A in Figure 2,
- Figure 6B: shows a detail Y of Figure 6A
- Figure 7A: shows a sectional top view along the line B-B in Figure 2,
- Figure 7B: shows a detail 2 of Figure 7A,
- Figure 8: shows a perspective view of an inhaler according to a second embodiment,
- Figure 9: shows a sectional side view of the inhaler according to the second embodiment,
- Figure 10: shows a perspective view of a nozzle block according to the second embodiment,
- Figure 11: shows a front view of the nozzle block of Figure 10,
- Figure 12: shows a perspective rear view of the nozzle block of Figure 10,
- Figure 13A: shows a sectional top view along the line A-A in Figure 9,
- Figure 13B: shows a detail Y' of Figure 13A,
- Figure 14A: shows a sectional top view along the line B-B in Figure 9 and
- Figure 14B: shows a detail Z' of Figure 14A.

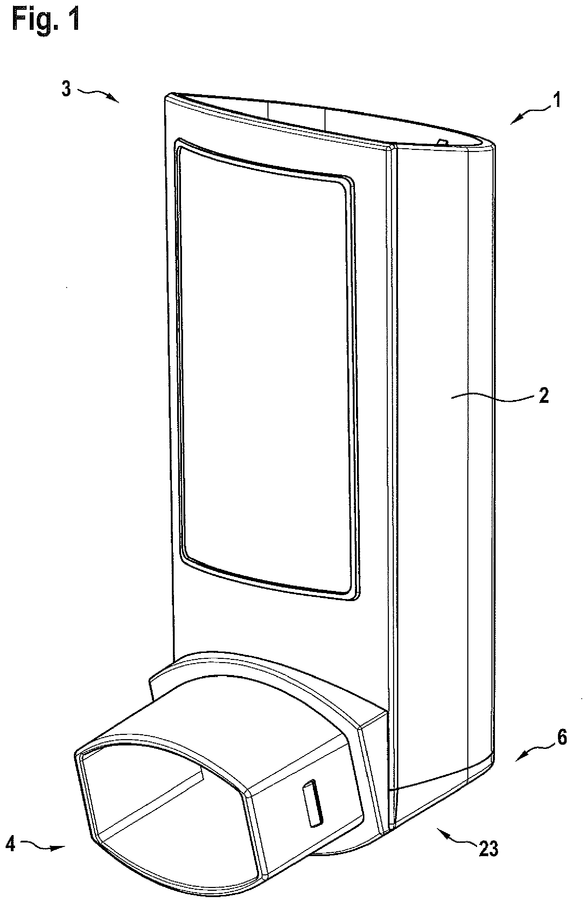

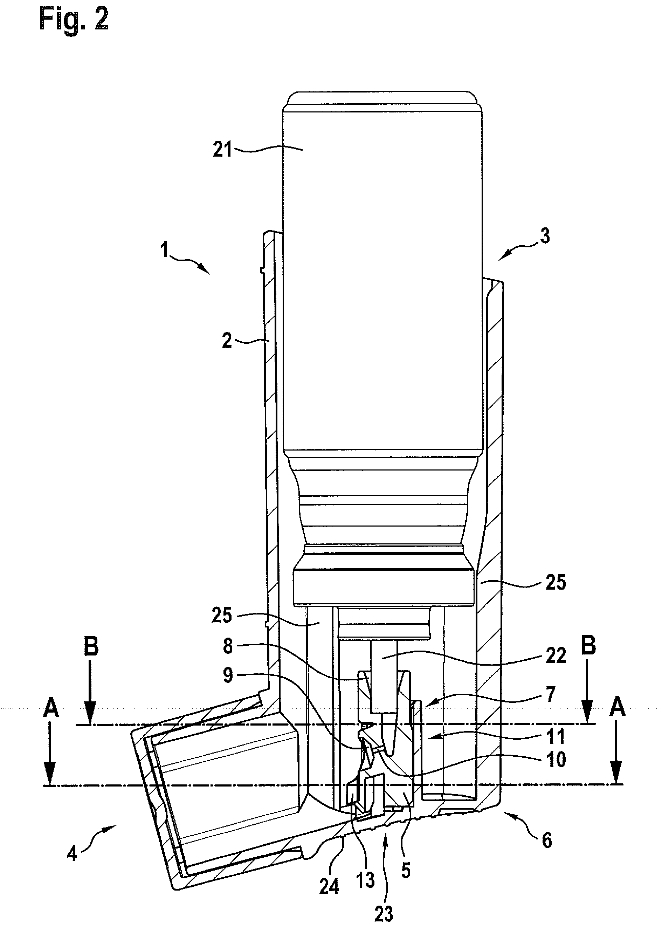

Figures 1 and 2 show an inhaler 1 with a hollow inhaler body 2 for retaining an aerosol container 21 with a dispensing valve 22 at a valve end of the container. The inhaler 1 comprises a first open end 3 sized and arranged to receive the aerosol container 21 with a dispensing valve 22 and a second open end 4 through which a dose of a medicament is dispensed as an aerosol. The second open end 4 is sized and arranged to be coupled to the mouth or nasal cavities of a patient (not shown). The inhaler body 2 has a bottom side 23 and comprises corrugations 24 thereon. The inhaler body 2 forms an angular hollow tubular body extending from the first open end 3 towards the second open end 4.

The inhaler body 2 further comprises ribs 25 which extend from the first open end 3 towards a bottom portion 6 of the inhaler body 2 and which are adapted to abut the aerosol container 21 upon its insertion into the inhaler body 2. Preferably, the ribs 25 are arranged on an insert (not shown) configured to be inserted into the inhaler body 2 through its first open end 3. Optionally, the insert may be exchangeable such that multiple inserts with different sizes of the ribs 25 may alternatively be inserted into the inhaler body 2 in order to accommodate aerosol containers 21 of different sizes.

The inhaler body 2 further comprises a nozzle block 5 which extends from the bottom portion 6 of the inhaler body 2 towards the first open end 3 and against which the dispensing valve 22 of the aerosol container 21 is depressed to release a dose of medicament. The nozzle block 5 has a fluid flow path 7 extending therethrough such that the dose of medicament being released from the valve 22 is passed to the nozzle block 5 and exits the nozzle block 5 as an aerosol in a direction towards the second open end 4 of the inhaler body 2.

The fluid flow path 7 of the nozzle block 5 is formed by a sump 8 configured to receive the dispensing valve 22 of the aerosol container, a nozzle 9 configured to aerosolize the medicament being released from the dispensing valve 22 and a channel 10 extending from the sump 8 to the nozzle 9.

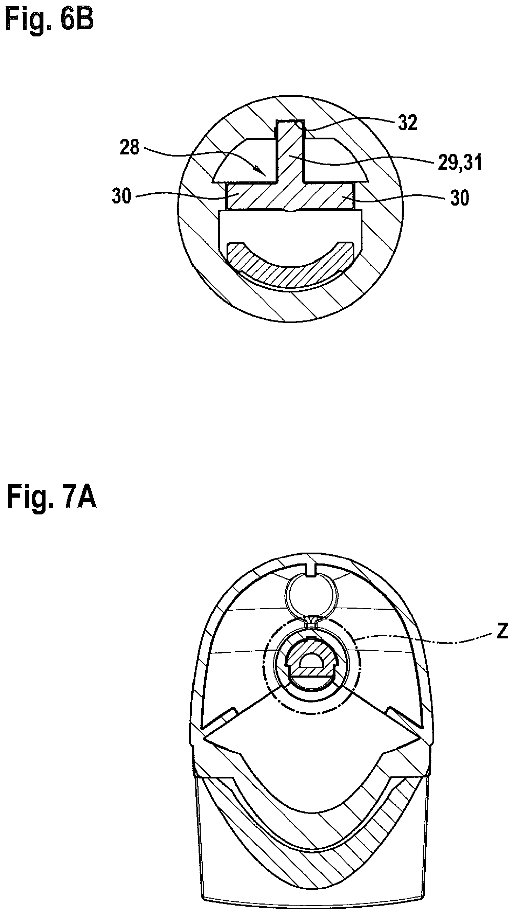

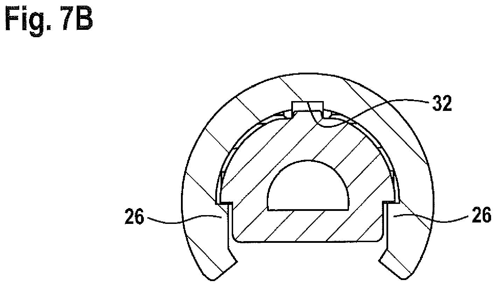

The nozzle block 5 is formed as a part separate from the inhaler body 2 and is adapted to be inserted through the first open end 3 of the inhaler body 2 into a receiving portion 11 at the bottom portion 6 of the inhaler body 2. The nozzle block 5 is connected to the bottom portion 6 of the inhaler body 2 via a form fitting connection. The receiving portion 11 of the bottom portion 6 has an essentially cylindrical shape and comprises opposing protrusions 26 (Figure 7B) which protrude radially inward.

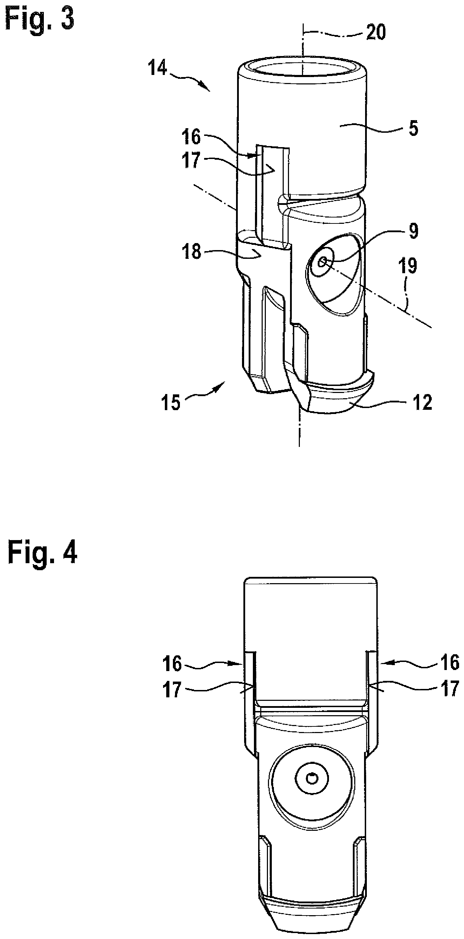

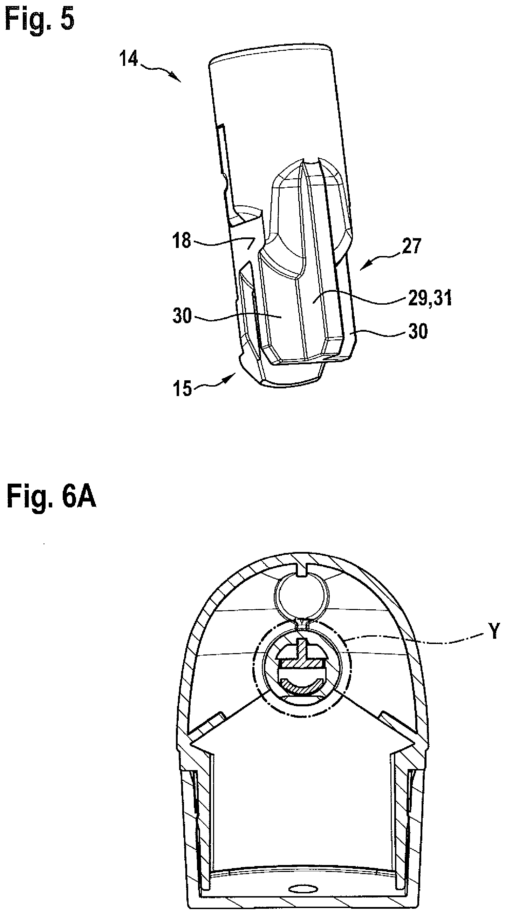

Figures 3 to 7A show the nozzle block 5 and its position in the receiving portion 11 in detail.

The nozzle block 5 has an at least partially cylindrical shape. It comprises a radially outward extending lower edge 12 which is adapted to engage with a slot 13 (Figure 2) in the receiving portion 11 of the bottom portion 6 to form the form fitting connecting between the nozzle block 5 and the inhaler body 2.

The nozzle block 5 has an upper portion 14, and a lower portion 15 positioned adjacent to one another. The nozzle block 5 comprises at its upper portion 14 opposing recesses 16, which each have a plane base surface 17, wherein the base surfaces 17 are arranged parallel to one another.

The opposing recesses 16 of the nozzle block 5 are configured to engage with the opposing protrusions 26 of the receiving portion 11 upon insertion of the nozzle block 5 into the receiving portion 11.

The nozzle block 5 has a supporting leg 27 for vertically supporting the nozzle block 5 in the receiving portion 11. The supporting leg 27 comprises a T-shaped cross sectional area 28 having a stem 29 and two opposing arms 30. The stem 29 forms a rib 31 which extends from the upper portion 14 along the lower portion 15. The rib 31 is adapted to engage with a vertically extending groove 32 in the receiving portion 11.

The nozzle block 5 further comprises at its lower portion 15 opposing plane side surfaces 18 which are arranged parallel to one another. The side surfaces 18 of the lower portion 15 are arranged parallel to a fist axis 19 defined by the channel 10 connecting the sump 8 and the nozzle 9 of the nozzle block 5 and are arranged parallel to a second axis 20 defined by the nozzle block 5 extending from the bottom portion 6 towards the first open end 3 of the inhaler body 2. The nozzle block 5 may be formed of transparent material.

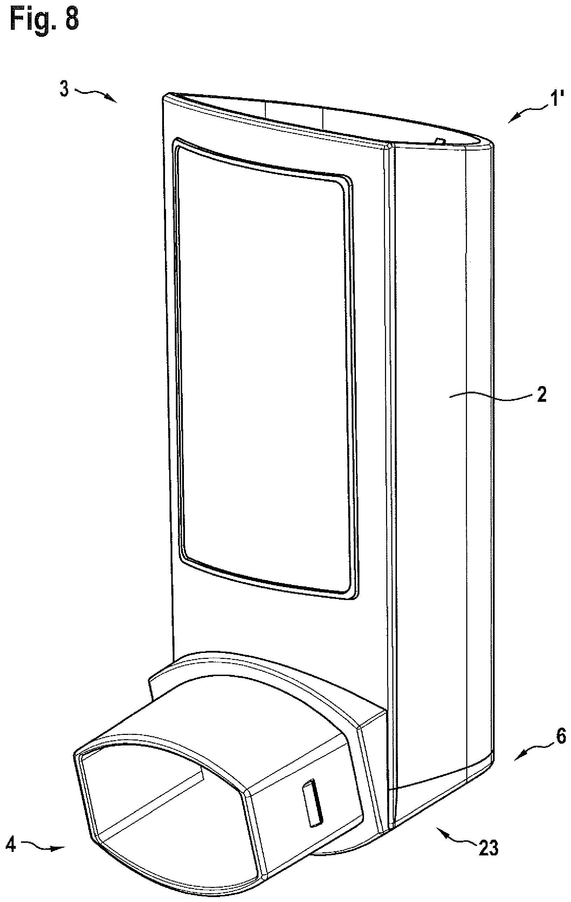

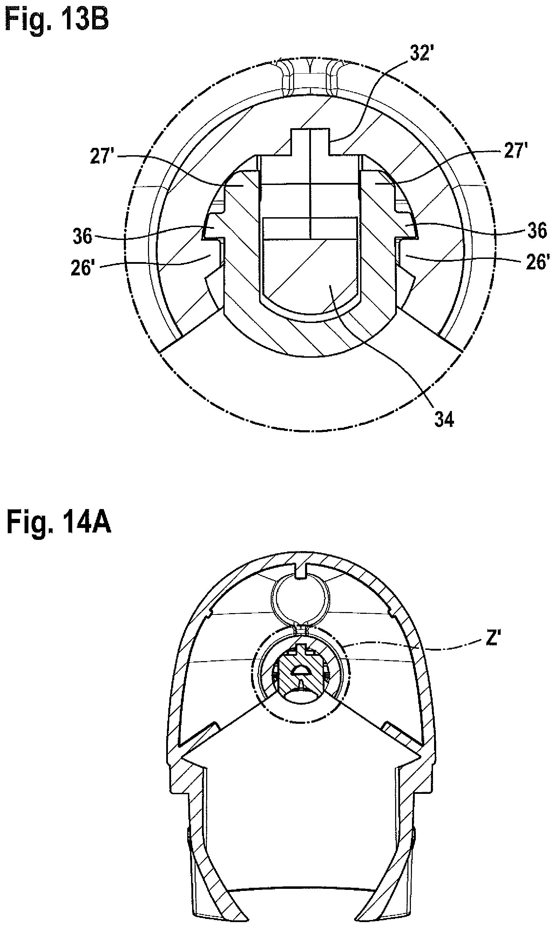

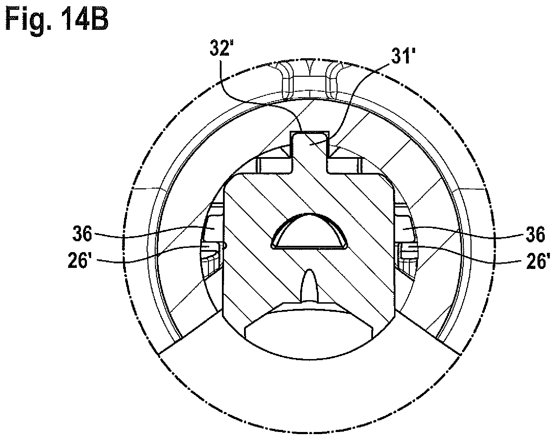

A second embodiment of the inhaler 1 will be described in the following with reference to Figures 8 to 14B.

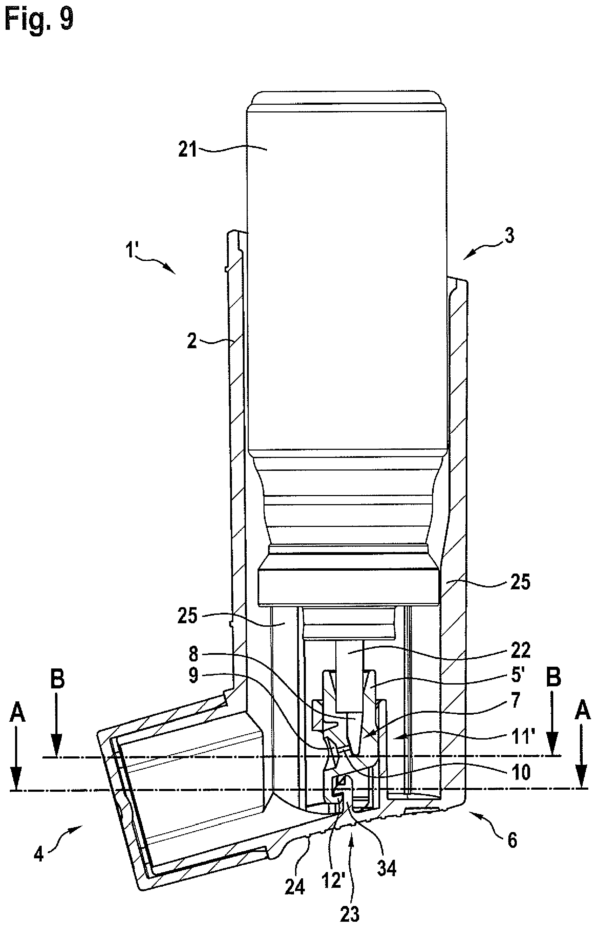

Figures 8 and 9 show an inhaler 1' with a hollow inhaler body 2 for retaining an aerosol container 21 with a dispensing valve 22 at a valve end of the container 21. The inhaler 1' comprises a first open end 3 sized and arranged to receive the aerosol container 21 with a dispensing valve 22 and a second open end 4 through which a dose of medicament is dispensed as an aerosol. The second open end 4 is sized and arranged to be coupled to the mouth on nasal cavities of a patient (not shown) . The inhaler body 2 has a bottom side 23 and comprises corrugations 24 thereon. The inhaler body 2 forms an angular hollow tubular body extending from the first open end 3 towards the second open end 4.

The inhaler body 2 further comprises ribs 25 which extend from the first open end 3 towards a bottom portion 6 of the inhaler body 2 and which are adapted to abut the aerosol container 21 upon its insertion into the inhaler body 2. Preferably the ribs 25 are arranged on an insert (not shown) configured to be inserted into the inhaler body 2 through its first open end 3. Optionally, the insert may be exchangeable such that multiple inserts with different sizes of the ribs 25 may alternatively be inserted into the inhaler body 2 in order to accommodate aerosol containers 21 of different sizes.

The inhaler body 2 further comprises a nozzle block 5' with an upper portion 14 and a lower portion 15 positioned adjacent to one another. The nozzle block 5' extends along a longitudinal axis 33 from the bottom portion 6 of the inhaler body 2 towards the first open end 3. The nozzle block 5' is configured such that a dose of a medicament is released upon depression of the dispensing valve 22 of the aerosol container 21 against the nozzle block 5'. The nozzle block 5' has a fluid flow path 7 extending therethrough such that the dose of medicament being released from the valve 22 is passed to the nozzle block 5' and exits the nozzle block 5' as an aerosol in a direction towards the second open end 4 of the inhaler body 2.

The fluid flow path 7 of the nozzle block 5' is formed by a sump 8 configured to receive the dispensing valve 22 of the aerosol container. A nozzle 9 is configured to aerosolize the dose of medicament being released from the dispensing valve 22 and a channel 10 extending from the sump 8 to the nozzle 9.

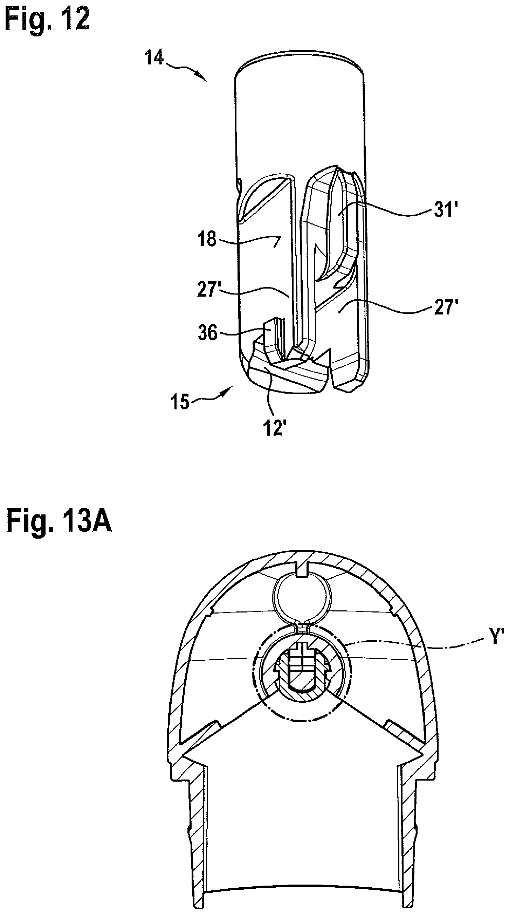

The nozzle block 5' is formed as apart separate from the inhaler body 2 and is adapted to be inserted through a first open end 3 of the inhaler body 2 into the receiving portion 11' at the bottom portion 6 of the inhaler body 2. The nozzle block 5' is connected to the bottom portion 6 of the inhaler body 2 via a form fitting connection. The receiving portion 11' of the bottom portion 6 has an essentially cylindrical shape and comprises opposing protrusions 26' (Figure 12b) which extend radially inward.

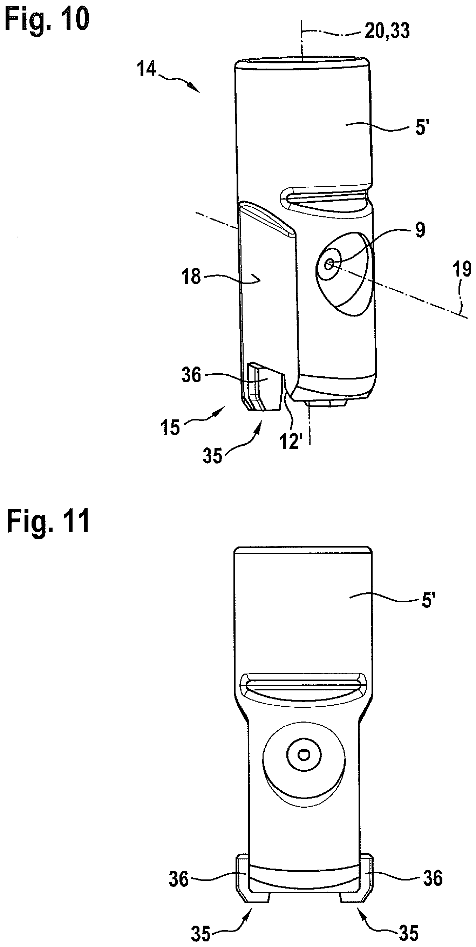

Figures 10 to 14B show the nozzle block 5' and its position in the receiving portion 11' in detail.

The nozzle block 5' has an at least partially cylindrical shape. It comprises a radially inward extending lower edge 12' which is adapted to engage with a vertically extending engagement member formed as a snap-in tongue 34 to form the form fitting connection between the nozzle block 5' and inhaler body 2. The snap-in tongue 34 is positioned in the receiving portion 11' of the bottom portion 6 and vertically extends therefrom towards the first open end 3 of the inhaler body 2.

The nozzle block 5' has two supporting legs 27' for vertically supporting the nozzle block 5' in the receiving portion 11'. Each of the supporting legs 27' has a lower end 35 with an alignment plate 36 protruding transversally to the longitudinal axis 33 of the nozzle block 5' from the corresponding supporting leg 27' in an outward direction. Each of the alignment plates 36 is configured to rest against the corresponding protrusion 26' of the receiving portion 11'. The alignment plates 36 protrude in opposing directions and the corresponding protrusions 26' of the receiving portion 11' oppose each other.

The nozzle block 5' further comprises a rib 31' which extends parallel to the supporting legs 27' from the upper portion 14 along the lower portion 15. The rip 31' is located between the supporting legs 27' and is adapted to engage with a vertically extending groove 32' in the receiving portion 11'.

The nozzle block 5' further comprises at its lower portion 15 opposing plain side surfaces 18 which are arranged parallel to one another. The side surface 18 of the lower portion 15 are arranged parallel to a first axis 19 defined by the channel 10 connecting the sump 8 and the nozzle 9 of the nozzle block 5' and arranged parallel to a second axis 20 defined by the nozzle block 5' extending from the bottom portion 6 towards the first open end 3 of the inhaler body 2. The nozzle block 5' may be formed of transparent material.

### Reference numerals

- 1,1': inhaler
- 2: inhaler body
- 3: first open end (inhaler body)
- 4: second open end (inhaler body)
- 5,5': nozzle block
- 6: bottom portion (inhaler body)
- 7: fluid flow path (nozzle block)
- 8: sump (nozzle block)
- 9: nozzle (nozzle block)
- 10: channel (nozzle block)
- 11,11': receiving portion (inhaler body)
- 12,12': lower edge (nozzle block)
- 13: slot (receiving portion)
- 14: upper portion (nozzle block)
- 15: lower portion (nozzle block)
- 16: recesses (nozzle block)
- 17: base surface (recesses)
- 18: side surface (nozzle block)
- 19: first axis (nozzle block)
- 20: second axis (nozzle block)
- 21: aerosol container
- 22: dispensing valve
- 23: bottom side (inhaler body)
- 24: corrugations (inhaler body)
- 25: ribs
- 26,26': opposing protrusions (receiving portion)
- 27,27': supporting leg (nozzle block)
- 28: cross sectional area (supporting leg)
- 29: stem (cross sectional area)
- 30: arms (cross sectional area)
- 31,31': rib (cross sectional area)
- 32,32': groove (receiving portion)
- 33: longitudinal axis (nozzle block)
- 34: snap-in-tongue (receiving portion)
- 35: lower end (supporting leg)
- 36: alignment plate (supporting leg)

## Claims

1. An inhaler having a hollow inhaler body (2) for retaining an aerosol container (21) with a dispensing valve (22) at a valve end of the container (21), the inhaler body (2) comprising:
- a first open end (3) sized and arranged to receive the aerosol container (21) with a dispensing valve (22);
- a second open end (4) through which a dose of medicament is dispensed as an aerosol, the second open end (4) being sized and arranged to be coupled to the mouth or nasal cavities of a patient;
- a nozzle block (5, 5') with an upper portion and a lower portion positioned adjacent to one another, the nozzle block (5, 5') extending along a longitudinal axis (33) from a bottom portion (6) of the inhaler body (2) towards the first open end (3), wherein the nozzle block (5, 5') is configured such that a dose of a medicament is released upon depression of the dispensing valve (22) of the aerosol container (21) against the nozzle block (5, 5'), the nozzle block (5, 5') having a fluid flow path (7) extending therethrough such that the dose of medicament being released from the valve (22) is passed to the nozzle block (5, 5') and exits the nozzle block (5, 5') as an aerosol in a direction towards the second open end (4) of the inhaler body (2);
wherein the nozzle block (5, 5') is formed as a part separate from the inhaler body (2),
wherein the bottom portion (6) of the inhaler body (2) comprises a receiving portion (11, 11') for receiving the nozzle block (5, 5')and
wherein the nozzle block (5, 5') is adapted to be inserted through the first open end (3) of the inhaler body (2) into the receiving portion (11, 11') of the bottom portion (6),
wherein the nozzle block (5') has two supporting legs (27') for vertically supporting the nozzle block (5') in the receiving portion (11'), wherein each of the supporting legs (27') has a lower end (35) with an alignment plate (36) protruding transversely to the longitudinal axis (33) of the nozzle block (5') from the corresponding supporting leg (27') in an outward direction and being configured to rest against a corresponding protrusion (26') of the receiving portion (11'), wherein the alignment plates (36) protrude in opposing directions and wherein the corresponding protrusions (26') of the receiving portion (11') oppose each other, and
**characterized in**
**that** the nozzle block (5') further has a rib (31') which extends parallel to the supporting legs (27') from the upper portion (14) along the lower portion (15) and is located between the supporting legs (27'), wherein the rib (31') is adapted to engage with a vertically extending groove (32') in the receiving portion (11').

2. Inhaler according to claim 1, **characterized in that** the nozzle block (5') is connected to the bottom portion (6) of the inhaler body (2) via a form fitting connection.

3. Inhaler according to claim 2, **characterized in that** the nozzle block (5') has an at least partially cylindrical shape, wherein the nozzle block (5') comprises a radially inward extending lower edge (12') which is adapted to engage with a vertically extending engagement member (34) in the receiving portion (11') of the bottom portion (6) to form the form fitting connecting.

4. Inhaler according to any of the preceding claims, **characterized in that** the receiving portion (11') of the bottom portion (6) has an essentially cylindrical shape, wherein the opposing protrusions (26') extend radially inward.

5. Inhaler according to any of the preceding claims, **characterized in that** the fluid flow path (7) of the nozzle block (5') is formed by a sump (8) configured to receive the dispensing valve (22) of the aerosol container (21), a nozzle (9) configured to aerosolize the dose of medicament being released from the dispensing valve (22) and a channel (10) extending from the sump (8) to the nozzle (9).

6. Inhaler according to any of the preceding claims, **characterized in that** the nozzle block (5') is formed of transparent material.

7. Inhaler according to any of the preceding claims, **characterized in that** the lower portion (15) of the nozzle block (5') comprises opposing plane side surfaces (18) which are arranged parallel to one another.

8. Inhaler according to claims 5 and 7, **characterized in that** the side surfaces (18) of the lower portion (15) are arranged parallel to a fist axis (19) defined by the channel (10) connecting the sump (8) and the nozzle (9) of the nozzle block (5') and are arranged parallel to a second axis (20) defined by the nozzle block (5') extending from the bottom portion (6) towards the first open end (3) of the inhaler body (2).

9. Inhaler according to any of the preceding claims, **characterized in that** the inhaler (1') comprises an insert configured to be inserted into the inhaler body (2) through its first open end (3), wherein the insert has ribs (25) which extend from the first open end (3) towards the bottom portion (6) of the inhaler body (2) and which are adapted to abut the aerosol container (21) upon insertion into the inhaler body (2).

10. Inhaler according to any of the preceding claims, **characterized in that** the inhaler body (2) has a bottom side (23), and comprises corrugations (24) thereon.

## Patentansprüche

1. Inhalator mit einem hohlen Inhalatorgehäuse (2) zum Halten eines Aerosolbehälters (21) mit einem Abgabeventil (22) an einem Ventilende des Behälters (21), wobei das Inhalatorgehäuse (2) umfasst:
- ein erstes offenes Ende (3), das so bemessen und angeordnet ist, dass es den Aerosolbehälter (21) mit einem Abgabeventil (22) aufnehmen kann;
- ein zweites offenes Ende (4), durch das eine Medikamentendosis als Aerosol abgegeben wird, wobei das zweite offene Ende (4) so bemessen und angeordnet ist, dass es mit dem Mund oder den Nasenhöhlen eines Patienten verbunden werden kann;
- einen Düsenblock (5, 5') mit einem oberen Abschnitt und einem unteren Abschnitt, die benachbart zueinander positioniert sind, wobei sich der Düsenblock (5, 5') entlang einer Längsachse (33) von einem Bodenabschnitt (6) des Inhalatorgehäuses (2) in Richtung des ersten offenen Endes (3) erstreckt, wobei der Düsenblock (5, 5') so konfiguriert ist, dass eine Medikamentendosis beim Niederdrücken des Abgabeventils (22) des Aerosolbehälters (21) gegen den Düsenblock (5, 5') abgegeben wird, wobei der Düsenblock (5, 5') einen Fluidströmungspfad (7) aufweist, der sich durch ihn hindurch erstreckt, so dass die Medikamentendosis, die von dem Ventil (22) freigegeben wird, zu dem Düsenblock (5, 5') geleitet wird und den Düsenblock (5, 5') als ein Aerosol in einer Richtung zu dem zweiten offenen Ende (4) des Inhalatorgehäuses (2) verlässt;
wobei der Düsenblock (5, 5') als ein vom Inhalatorgehäuse (2) getrenntes Teil ausgebildet ist,
wobei der Bodenabschnitt (6) des Inhalatorgehäuses (2) einen Aufnahmeabschnitt (11, 11') zur Aufnahme des Düsenblocks (5, 5') aufweist und
wobei der Düsenblock (5, 5') geeignet ist, durch das erste offene Ende (3) des Inhalatorgehäuses (2) in den Aufnahmeabschnitt (11, 11') des Bodenabschnitts (6) eingeführt zu werden,
wobei der Düsenblock (5') zwei Stützbeine (27') zum vertikalen Stützen des Düsenblocks (5') in dem Aufnahmeabschnitt (11') aufweist, wobei jedes der Stützbeine (27') ein unteres Ende (35) mit einer Ausrichtungsplatte (36) aufweist, die quer zu der Längsachse (33) des Düsenblocks (5') von dem entsprechenden Stützbein (27') in einer nach außen gerichteten Richtung vorsteht und so gestaltet ist, dass sie an einem entsprechenden Vorsprung (26') des Aufnahmeabschnitts (11') anliegt, wobei die Ausrichtungsplatten (36) in entgegengesetzte Richtungen vorstehen und wobei die entsprechenden Vorsprünge (26') des Aufnahmeabschnitts (11') einander gegenüberliegen, und dardurch gekennzeichnet, dass
der Düsenblock (5') ferner eine Rippe (31') aufweist, die sich parallel zu den Stützbeinen (27') von dem oberen Abschnitt (14) entlang des unteren Abschnitts (15) erstreckt und zwischen den Stützbeinen (27') angeordnet ist, wobei die Rippe (31') so angepasst ist, dass sie in eine sich vertikal erstreckende Nut (32') in dem Aufnahmeabschnitt (11') eingreift.

2. Inhalator nach Anspruch 1, **dadurch gekennzeichnet, dass** der Düsenblock (5') über eine formschlüssige Verbindung mit dem Bodenabschnitt (6) des Inhalatorgehäuses (2) verbunden ist.

3. Inhalator nach Anspruch 2, **dadurch gekennzeichnet, dass** der Düsenblock (5') eine zumindest teilweise zylindrische Form aufweist, wobei der Düsenblock (5') eine sich radial nach innen erstreckende untere Kante (12') aufweist, die so angepasst ist, dass sie mit einem sich vertikal erstreckenden Eingriffselement (34) in dem Aufnahmeabschnitt (11') des Bodenabschnitts (6) in Eingriff kommt, um die formschlüssige Verbindung zu bilden.

4. Inhalator nach einem der vorhergehenden Ansprüche, **dadurch gekennzeichnet, dass** der Aufnahmeabschnitt (11') des Bodenabschnitts (6) eine im Wesentlichen zylindrische Form aufweist, wobei sich die gegenüberliegenden Vorsprünge (26') radial nach innen erstrecken.

5. Inhalator nach einem der vorhergehenden Ansprüche, **dadurch gekennzeichnet, dass** der Fluidströmungspfad (7) des Düsenblocks (5') durch einen Sumpf (8), der so konfiguriert ist, dass er das Abgabeventil (22) des Aerosolbehälters (21) aufnimmt, eine Düse (9), die so konfiguriert ist, dass sie die aus dem Abgabeventil (22) abgegebene Medikamentendosis vernebelt, und einen Kanal (10) gebildet ist, der sich von dem Sumpf (8) zu der Düse (9) erstreckt.

6. Inhalator nach einem der vorhergehenden Ansprüche, **dadurch gekennzeichnet, dass** der Düsenblock (5') aus transparentem Material gebildet ist.

7. Inhalator nach einem der vorhergehenden Ansprüche, **dadurch gekennzeichnet, dass** der untere Abschnitt (15) des Düsenblocks (5') gegenüberliegende ebene Seitenflächen (18) aufweist, die parallel zueinander angeordnet sind.

8. Inhalator nach einem der Ansprüche 5 und 7, **dadurch gekennzeichnet, dass** die Seitenflächen (18) des unteren Abschnitts (15) parallel zu einer ersten Achse (19) angeordnet sind, die durch den Kanal (10) definiert ist, der den Sumpf (8) und die Düse (9) des Düsenblocks (5') verbindet, und parallel zu einer zweiten Achse (20) angeordnet sind, die durch den Düsenblock (5') definiert ist und sich vom Bodenabschnitt (6) zum ersten offenen Ende (3) des Inhalatorgehäuses (2) erstreckt.

9. Inhalator nach einem der vorhergehenden Ansprüche, **dadurch gekennzeichnet, dass** der Inhalator (1') einen Einsatz umfasst, der so konfiguriert ist, dass er durch das erste offene Ende (3) des Inhalatorgehäuse (2) in dieses eingesetzt werden kann, wobei der Einsatz Rippen (25) aufweist, die sich von dem ersten offenen Ende (3) in Richtung des Bodenabschnitts (6) des Inhalatorgehäuses (2) erstrecken und die so angepasst sind, dass sie beim Einsetzen in das Inhalatorgehäuse (2) an dem Aerosolbehälter (21) anliegen.

10. Inhalator nach einem der vorhergehenden Ansprüche, **dadurch gekennzeichnet, dass** das Inhalatorgehäuse (2) eine Unterseite (23) hat und Riffelungen (24) darauf aufweist.

## Revendications

1. Inhalateur comportant un corps d'inhalateur creux (2) destiné à retenir un récipient pressurisé (21) comportant une vanne distributrice (22) à une extrémité de vanne du récipient (21), le corps d'inhalateur (2) comprenant :
- une première extrémité ouverte (3) dimensionnée et conçue pour recevoir le récipient pressurisé (21) pourvu d'une vanne distributrice (22) ;
- une deuxième extrémité ouverte (4) par laquelle une dose de médicament est délivrée sous forme d'aérosol, la deuxième extrémité ouverte (4) étant dimensionnée et conçue pour être accouplée à la cavité buccale ou nasale d'un patient ;
- un bloc de buse (5, 5') comportant une partie supérieure et une partie inférieure adjacentes l'une à l'autre, le bloc de buse (5, 5') s'étendant le long d'un axe longitudinal (33), d'une partie basse (6) du corps d'inhalateur (2) vers la première extrémité ouverte (3), dans lequel le bloc de buse (5, 5') est configuré de telle manière qu'une dose de médicament est libérée lorsque la vanne distributrice (22) du récipient pressurisé (21) est pressée contre le bloc de buse (5, 5'), le bloc de buse (5, 5') comportant un chemin d'écoulement fluide (7) qui s'étend à travers celui-ci de telle manière que la dose de médicament qui est libérée par la vanne (22) passe dans le bloc de buse (5, 5') et sort du bloc de buse (5, 5') sous forme d'aérosol dans une direction orientée vers la deuxième extrémité ouverte (4) du corps d'inhalateur (2) ;
dans lequel le bloc de buse (5, 5') est formé en tant que pièce distincte du corps d'inhalateur (2),
dans lequel la partie basse (6) du corps d'inhalateur (2) comprend une partie réceptrice (11, 11') destinée à recevoir le bloc de buse (5, 5'), et
dans lequel le bloc de buse (5, 5') est adapté pour être inséré, à travers la première extrémité ouverte (3) du corps d'inhalateur (2), dans la partie réceptrice (11, 11') de la partie basse (6),
dans lequel le bloc de buse (5') comporte deux jambes de support (27') destinées à supporter verticalement le bloc de buse (5') dans la partie réceptrice (11'), dans lequel chacune des jambes de support (27') a une extrémité inférieure (35) avec une plaquette d'alignement (36) faisant saillie transversalement par rapport à l'axe longitudinal (33) du bloc de buse (5') depuis la jambe de support (27') correspondante dans une direction orientée vers l'extérieur et étant configurée pour prendre appui sur une protubérance correspondante (26') de la partie réceptrice (11'), dans lequel les plaquettes d'alignement (36) font saillie dans des directions opposées et dans lequel les protubérances correspondantes (26') de la partie réceptrice (11') sont opposées l'une à l'autre, et
**caractérisé en ce que** le bloc de buse (5') comporte en outre une nervure (31') qui s'étend parallèlement aux jambes de support (27') depuis la partie supérieure (14) le long de la partie inférieure (15) et qui est située entre les jambes de support (27'), dans lequel la nervure (31') est adaptée pour se mettre en prise avec une rainure s'étendant verticalement (32') dans la partie réceptrice (11').

2. Inhalateur selon la revendication 1, **caractérisé en ce que** le bloc de buse (5') est raccordé à la partie basse (6) du corps d'inhalateur (2) par emmanchement à force.

3. Inhalateur selon la revendication 2, **caractérisé en ce que** le bloc de buse (5') a une forme au moins partiellement cylindrique, le bloc de buse (5') comprenant un bord inférieur s'étendant radialement vers l'intérieur (12') qui est adapté pour se mettre en prise avec un élément de mise en prise s'étendant verticalement (34) dans la partie réceptrice (11') de la partie basse (6) pour former le raccordement par emmanchement à force.

4. Inhalateur selon l'une quelconque des revendications précédentes, **caractérisé en ce que** la partie réceptrice (11') de la partie basse (6) a une forme essentiellement cylindrique, dans laquelle les protubérances opposées (26') s'étendent radialement vers l'intérieur.

5. Inhalateur selon l'une quelconque des revendications précédentes, **caractérisé en ce que** le chemin d'écoulement fluide (7) du bloc de buse (5') est formé par un siphon (8) configuré pour recevoir la vanne distributrice (22) du récipient pressurisé (21), une buse (9) configurée pour vaporiser la dose de médicament qui est libérée par la vanne distributrice (22) et un canal (10) s'étendant du siphon (8) à la buse (9).

6. Inhalateur selon l'une quelconque des revendications précédentes, **caractérisé en ce que** le bloc de buse (5') est fait d'un matériau transparent.

7. Inhalateur selon l'une quelconque des revendications précédentes, **caractérisé en ce que** la partie inférieure (15) du bloc de buse (5') comprend des surfaces latérales planes opposées (18) qui sont parallèles entre elles.

8. Inhalateur selon les revendications 5 et 7, **caractérisé en ce que** les surfaces latérales (18) de la partie inférieure (15) sont disposées parallèlement à un premier axe (19) défini par le canal (10) qui relie le siphon (8) et la buse (9) du bloc de buse (5') et sont disposées parallèlement à un deuxième axe (20) défini par le bloc de buse (5') s'étendant depuis la partie basse (6) vers la première extrémité ouverte (3) du corps d'inhalateur (2).

9. Inhalateur selon l'une quelconque des revendications précédentes, **caractérisé en ce que** l'inhalateur (1') comprend un insert configuré pour être inséré dans le corps d'inhalateur (2) à travers sa première extrémité ouverte (3), l'insert comportant des nervures (25) qui s'étendent depuis la première extrémité ouverte (3) vers la partie basse (6) du corps d'inhalateur (2) et qui sont adaptées pour être en appui contre le récipient pressurisé (21) lors de l'insertion dans le corps d'inhalateur (2).

10. Inhalateur selon l'une quelconque des revendications précédentes, **caractérisé en ce que** le corps d'inhalateur (2) a une face inférieure (23), et comprend des cannelures (24) sur cette dernière.
